# EUROPÄISCHE PATENTSCHRIFT

(11) **EP 3 782 683 B1**
(45) Veröffentlichungstag und Bekanntmachung des Hinweises auf die Patenterteilung: **13.08.2025**
(21) Anmeldenummer: 20020380.0
(22) Anmeldetag: 21.08.2020
(51) Int. Cl.: A61M 16/04, A61M 16/20, A61M 16/00, A61M 16/08

(54) **SYSTEM ZUR ATEMGASVERSORGUNG BEI DER BEATMUNG MIT EINEM TUBUS**
BREATHING GAS SUPPLY SYSTEM FOR VENTILATION WITH A TUBE
SYSTÈME D'ALIMENTATION EN GAZ RESPIRATOIRE LORS DE LA RESPIRATION À L'AIDE D'UN TUBE

(30) Priorität: 23.08.2019 DE 102019005943
(43) Veröffentlichungstag der Anmeldung: 24.02.2021
(73) Patentinhaber: Löwenstein Medical Technology S.A., 2557 Luxembourg (LU)
(72) Erfinder: Kremeier, Peter, 76149 Karlsruhe (DE); Pulletz, Sven, 49090 Osnabrück (DE)
(74) Vertreter: Löwenstein Medical IP

(56) Entgegenhaltungen:
- DE-C1- 19 826 690
- US-A- 4 630 606
- US-A1- 2003 000 526
- US-A1- 2018 021 540

## Beschreibung

Die ventilatorassoziierte Pneumonie (VAP) ist die häufigste nosokomiale Infektion bei Beatmungspatienten auf der Intensivstation. Vielfach wird diese durch Erreger aus dem oberen Respirationstrakt verursacht, welche aufgrund von Mikroaspirationen am Tubus-Ballon vorbei in die Lunge gelangen. Die ventilatorassoziierte Pneumonie erhöht die Sterblichkeitsrate zusätzlich um ca. 10%, verlängert die Beatmungsdauer und verursacht zusätzliche Kosten. US 2003/0000526 A1 offenbart ein Verfahren zur Steuerung des Atemgasflusses für eine Beatmung mit einem Beatmungstubus und aufblasbaren Cuff, wobei der Atemgasfluß in Abhängigkeit eines erfassten intra-Cuffdrucks gesteuert wird. US 2018/0021540 A1 offenbart ein Ventil für einen Cuff eines Trachealtubus.

DE3435900 bezieht sich auf eine Einrichtung zum intermittierenden Aufblasen eines Cuffs für ein Patienten-Beatmungsgerät.

Als eine Maßnahme innerhalb eines Maßnahmenbündels zur Reduzierung des VAP-Risikos gilt die kontinuierliche Überwachung und Steuerung des geblockten Ballons des Endotrachealtubus bzw. der Trachealkanüle. Die auf Intensivstationen angewendete intermittierende Ballon-Kontrolle mithilfe eines Manometers ist weit verbreitet. Damit sollen Komplikationen im Zusammenhang mit einem zu niedrigen oder zu hohen Ballondruck vermieden werden. Obwohl durch diese Methode theoretisch eine Verringerung der Komplikationen erreicht werden könnte, ist sie zur dauerhaften Aufrechterhaltung und kontinuierlichen Überwachung einer adäquaten Tubusblockierung unzureichend geeignet. Die wichtigste Funktion des Trachealtubus-Ballons besteht darin, den extraluminalen Atemweg gegen Luft und eine Flüssigkeitsleckage zu verschließen, damit eine wirksame maschinelle Beatmung mit positivem Druck möglich ist, und um eine Mikroaspiration von kontaminiertem subglottischem Sekret zu vermeiden. Ein zu geringer Druck im Ballon sorgt für eine Mikroaspiration. Ein zu hoher Druck für Läsionen.

### Aufgabe

Die erfindungsgemäße Aufgabe ist es ein verbessertes und sicheres System zur Atemgasversorgung mit einem Trachealtubus-Ballon anzugeben.

Die Aufgabe wird gelöst durch ein System zur Atemgasversorgung umfassend ein Beatmungsgerät eine Atemgasquelle , eine Steuereinheit , ein Drucksteuermodul und/oder ein Flusssteuermodul und einen Speicher, wobei ebenfalls eine Drucksensoreinrichtung und eine Flusssensoreinrichtung und eine Anwenderschnittstelle zur Vorgabe von Parametern der Atemgasversorgung oder zum Austausch von Daten umfasst sind, wobei das Beatmungsgerät zumindest einen Schlauchanschluss aufweist, an den der Atemgasschlauch adaptiert werden kann und ein Patienteninterface als Schnittstelle zum Patienten und zumindest einen Druckanschluss für einen zusätzlichen Druckschlauch wobei als Patienteninterface ein Tubus verwendet ist, der einen Ballon aufweist, der über den Druckanschluss und den daran adaptierten Druckschlauch definiert mit Druckgas befüllbar ist. Erfindungsgemäß variiert die Steuereinheit den Ballondruck entsprechend dem Druck der Inspiration und der Exspiration. Das erfindungsgemäße System ist auch dadurch gekennzeichnet, dass das Patienteninterface als ein Tubus für die Luftröhre oder für die Speiseröhre oder als ein Blasenkatheter ausgebildet ist.

Das erfindungsgemäße System ist alternativ dadurch gekennzeichnet, dass die Steuereinheit eingerichtet und ausgebildet ist die Atemgasquelle, unter Verwendung des Drucksteuermoduls oder des Flusssteuermoduls, zur Vorgabe eines Atemgasdruckes und/oder eines Atemgasflusses anzusteuern und das Atemgas am Schlauchanschluss bereitgestellt und über den Atemgasschlauch zu einem Patienten geleitet wird.

Das erfindungsgemäße System ist ergänzend dadurch gekennzeichnet, dass die Steuereinheit eingerichtet und ausgebildet ist zusätzlich zumindest zeitweise den Anfeuchter oder den Vernebler und die Heizung zur Erwärmung und Anfeuchtung des Atemgases oder eine Sauerstoffquelle zu aktivieren.

Das erfindungsgemäße System ist auch dadurch gekennzeichnet, dass zusätzlich ein Pulsoximeter oder ein Co -Messgerät adaptiert ist, welches dann mit dem System kommuniziert und/oder von ihm mit Energie versorgt wird und wobei die Steuereinheit eingerichtet und ausgebildet ist die Messwerte dieser Geräte oder dem Anwender an der Schnittstelle bereitzustellen und/oder ein automatischer Algorithmus die richtige Flussrate und die Messwerte dieser Geräte oder für die Aktivierung oder Steuerung der Sauerstoffquelle und/oder bei der Aktivierung oder Steuerung der Atemgasquelle zu nutzen.

Das erfindungsgemäße System ist alternativ auch dadurch gekennzeichnet, dass das Patienteninterface als Endotrachealtubus mit trachealem Ende und mit einem distalen Ende, mit Tubuskonnektor zum Anschluss an den Atemgasschlauch und einem durchgehenden Lumen für die Atemgase ausgeführt ist, wobei nahe am trachealen Ende der aufblasbare Ballon ausgebildet ist, wobei der Ballon über eine Zuführleitung , die über den Druckschlauch mit dem Druckanschluss des Beatmungsgerätes verbunden ist, mit Druckgas aus der Atemgasquelle oder einer Druckgasquelle befüllbar ist.

Das erfindungsgemäße System ist ergänzend auch dadurch gekennzeichnet, dass die Steuereinheit auch eingerichtet und ausgebildet ist die Atemgasquelle , unter Verwendung des Drucksteuermoduls so anzusteuern, dass ein Druckgas über eine Leitung zum Druckanschluss des Beatmungsgerätes geführt wird, wobei zumindest ein Druckanschluss vorhanden, und jedem Druckanschluss ein Drucksensor zugeordnet ist, der den Druck des Gases in dem Druckanschluss bestimmen kann und/oder jedem Druckanschluss auch ein Ventil zugeordnet ist, welches eingerichtet ist, eine Druckgasströmung zu dem Druckanschluss freizugeben oder zu sperren.

Das erfindungsgemäße System ist optional auch dadurch gekennzeichnet, dass ein Anwender über die Schnittstelle den Ballondruck festlegen kann und die Steuereinheit über den Drucksensor und den Druckregler den gewünschten Druck im Ballon einstellt, selbsttätig nachregelt und den Ballondruck (Pbal) als Signal der Steuerung des Beatmungsgerätes zur Verfügung stellt, wobei der Ballondruck (Pbal) beispielsweise entsprechend dem Druck der Inspiration und Exspiration variiert wird.

Das erfindungsgemäße System ist beispielsweise auch dadurch gekennzeichnet, dass der Druckanschluss des Beatmungsgerätes ein Verbindungsmittel aufweist, zur lösbaren Verbindung mit dem Beatmungsgerät, und ein inneres Lumen und eine Druckgasleitung im inneren Lumen, sowie einen männlichen Anschluss a, der zumindest einen konischen Abschnitt aufweist, der zur lösbaren Verbindung mit der weiblichen Aufnahme des Druckadapters ausgebildet ist.

Das erfindungsgemäße System ist ergänzend auch dadurch gekennzeichnet, dass ein Druckadapter umfasst ist, welcher einen männlichen Luer-Anschluß aufweist, der an einem kurzen Schlauchabschnitt angeordnet ist, und der eine weibliche Aufnahme für den männlichen Druckanschluss des Beatmungsgerätes aufweist, wobei ein Rückschlagventil in dem Druckadapter angeordnet ist, das in einer Schließstellung vorgespannt ist und dessen Ventilkörper durch den Druckanschluss in eine Öffnungsstellung bewegt werden kann.

Das erfindungsgemäße System ist alternativ auch dadurch gekennzeichnet, dass ein Druckschlauch umfasst ist, der an einem Ende einen Luer-Lock Anschluss aufweist und an dem anderen Ende zumindest einen Druckadapter aufweist, der auf den Druckanschluss des Beatmungsgerätes gesteckt werden kann, wobei ein Rückschlagventil in dem Druckadapter angeordnet ist, das in einer Schließstellung vorgespannt ist und dessen Ventilkörper, bei der Verbindung mit dem Druckanschluss, durch den Druckanschluss in eine Öffnungsstellung bewegt wird.

Das erfindungsgemäße System ist auch dadurch gekennzeichnet, dass der Druckadapter einen männlichen Luer-Anschluß aufweist, der an einem Schlauchabschnitt angeordnet ist, und der zur Verbindung mit einem weiblichen Luer-Anschluss eines Druckschlauches angepasst ist, wobei der Druckadapter zudem eine weibliche Aufnahme für den männlichen Druckanschluss des Beatmungsgerätes aufweist.

Das erfindungsgemäße System ist zudem dadurch gekennzeichnet, dass im Bereich der weiblichen Aufnahme ein Rückschlagventil angeordnet ist, welches durch eine Feder in einer Schließstellung vorgespannt ist, wobei der Ventilkörper des Rückschlagventils durch den Druckanschluss in eine Öffnungsstellung bewegbar ist.

Das erfindungsgemäße System ist auch dadurch gekennzeichnet, dass der Druckanschluss eine Druckgasleitung I aufweist die Druckgas leitet und auch dazu dient, den Ventilkörper in eine Öffnungsstellung zu bewegen, wenn der Druckadapter mit dem Druckanschluss verbunden wird.

Das erfindungsgemäße System ist alternativ dadurch gekennzeichnet, dass der Ventilkörper eine Kontaktfläche aufweist, gegen die die (Spitze der) Druckgasleitung gedrängt wird, wenn der männliche Druckanschluss in die weibliche Aufnahme geschoben wird.

Der männliche Druckanschluss drückt dann (mittels der Druckgasleitung I) den Ventilkörper gegen die Federkraft in die Offenstellung, die einen Gasfluss aus dem männlichen Druckanschluss in den Druckadpter (oder den Druckschlauch) ermöglicht.

Das erfindungsgemäße System ist beispielsweise dadurch gekennzeichnet, dass die weibliche Aufnahme aus einem weichen oder elastomeren Material gefertigt ist und ein Innenlumen aufweist, welches dem Außendurchmesser des männlichen Druckanschlusses des Beatmungsgerätes so angepasst ist, dass der männliche Druckanschluss in die weibliche Aufnahme eingeführt werden kann und eine dichtende und klemmende Verbindung zwischen beiden sicherstellt ist.

Der Druckadapter kann so auf den Druckanschluss gesetzt werden und führt zu einer abgedichteten Strömungsverbindung von Druckgas aus dem Druckanschluss in den Druckadapter.

Das erfindungsgemäße System ist alternativ dadurch gekennzeichnet, dass der Druck des Ballons dynamisch an die jeweilige Drucksituation in der Inspiration angepasst wird. Gerade bei Patienten mit hohen Beatmungsdrücken kann hierdurch sowohl eine adäquate Blockung im Endotrachealtubus bzw. in der Trachealkanüle in der Inspiration sichergestellt werden, Das erfindungsgemäße System ist auch dadurch gekennzeichnet, dass der Druck des Ballons dynamisch an die jeweilige Drucksituation in der Exspiration angepasst wird. als auch die notwendige Entlastung für die Durchblutung der Trachealschleimhaut in der Exspirationsphase gewährleistet werden.

Das erfindungsgemäße System ist alternativ auch dadurch gekennzeichnet, dass die Steuereinheit auch eingerichtet und ausgebildet ist die Atemgasquelle , unter Verwendung des Drucksteuermoduls so anzusteuern, dass Druckgas über eine Leitung zum Druckanschluss des Beatmungsgerätes geführt wird, wobei dem Druckanschluss ein Drucksensor zugeordnet ist, der den Druck des Gases in dem Druckanschluss bestimmt, wobei der Druck des Gases in dem Druckanschluss dem Druck im Ballon (Pbal) im Wesentlichen entspricht, wenn eine Druckgasleitende Verbindung zwischen dem Ballon und dem Druckgasanschluss hergestellt ist.

Das erfindungsgemäße System ist ergänzend dadurch gekennzeichnet, dass die Steuereinheit einen Druckregler aufweist oder diesen ansteuert, um den gewünschten Druck im Ballon einzustellen und den Ballondruck (Pbal) als beispielsweise digitalisiertes Signal der Steuereinheit zur Verfügung stellt.

Das erfindungsgemäße System ist alternativ auch dadurch gekennzeichnet, dass die Steuereinheit den Ballondruck (Pbal) entsprechend dem Druck der Inspiration und der Exspiration variiert.

Das erfindungsgemäße System ist auch dadurch gekennzeichnet, dass der gemessene Ballondruck als zusätzliche Druckkurve auf dem Beatmungsgerät angezeigt wird.

Die Aufgabe wird auch gelöst durch ein Beatmungsgerät eingerichtet und ausgebildet zur Verwendung in einem erfindungsgemäßen System.

Die Aufgabe wird auch gelöst durch einen Druckadapter eingerichtet und ausgebildet zur Verwendung in einem erfindungsgemäßen System. Der Druckadapter weist einen männlichen Luer-Anschluß aufweist, der an einem kurzen Schlauchabschnitt angeordnet ist, und der eine weibliche Aufnahme für den männlichen Druckanschluss des Beatmungsgerätes aufweist, wobei ein Rückschlagventil in dem Druckadapter angeordnet ist, welches in einer Schließstellung vorgespannt ist und dessen Ventilkörper durch den Druckanschluss in eine Öffnungsstellung bewegt werden kann.

Die Aufgabe wird auch gelöst durch einen Druckschlauch eingerichtet und ausgebildet zur Verwendung in einem erfindungsgemäßen System. Der Druckschlauch weist an einem Ende einen Luer-Lock Anschluss auf und an dem anderen Ende zumindest einen Druckadapter auf, der auf den Druckanschluss gesetzt werden kann, wobei ein Rückschlagventil in dem Druckadapter angeordnet ist, welches in einer Schließstellung vorgespannt ist und dessen Ventilkörper durch den Druckanschluss in eine Öffnungsstellung bewegt werden kann.

Die Aufgabe wird auch gelöst durch einen Druckschlauch 25 mit einem Druckadapter 26 eingerichtet und ausgebildet zur Verwendung in einem System nach zumindest einem der vorhergehenden Ansprüche.

Für den Ballondruck sollte in etwa ein Wert von 25 cm H2O aufrechterhalten werden, da die Mikroaspiration bei einem Wert von unter 20 cm H2O auftritt und das Risiko von ischämischen Läsionen der Trachea aufgrund der Minderdurchblutung der Trachealschleimhaut bei einem Druck von über 30 cmH2O eine größere Rolle spielt.

Die Erfindung bietet folgende Vorteile:
- Aufrechterhaltung und Überwachung eines durch den Anwender vorgegebenen Ballondrucks
- Zwei Betriebsarten: Gleichbleibende Regelung des Ballons (statisch) oder automatische Anpassung an den Plateaudruck
- Erkennung von defekten Ballonmanschetten (Alarm) und Leckagen
- Algorithmus zur Hustenerkennung (Ballonanpassung)
- Modus zur zeitweiligen Überblockung des Ballons (z.B. bei Mundpflege, subglottischen Spülungen, Bauchlagerung, ...)
- Modus für die Leerung des Ballons (Extubation)
   • Überwachung von Druckwerten des Ballons (PBallon), Zielwerte = 20-30 cmH2O
- da zu geringer Ballondruck < 20 cmH2O -> Mikroaspiration
- da dauerhaft zu hoher Ballondruck > 30 cmH2O -> Ischämie der Trachealschleimhaut

Die Funktionsweise des Ballons kann an die unterschiedlichen Bedürfnisse angepasst werden. Zur Überwachung und Optimierung des Ballons stehen zwei Ballon-Modi zur Verfügung. Die Verfügbarkeit der Ballon-Modi und die Regelung des automatischen Ballon-Modus können auf der Konfigurationsebene des Beatmungsgerätes (gemäß den Abteilungsvorgaben) eingestellt werden

Mit dem "statischen" Ballon-Modus gibt der Anwender einen definierten Ballondruck (PBallon konstant) vor, welcher kontinuierlich überwacht und optimiert wird.

Der kontinuierlich gemessene Ballondruck wird als zusätzliche Druckkurve angezeigt und kann mit entsprechenden Alarmen für Leckage, defekte Tubusblockmanschette und Drucküberwachung versehen werden.

Im "automatischen" Modus adaptiert sich die Blockung des Ballons an die jeweilige Drucksituation in der Inspiration. Gerade bei Patienten mit hohen Beatmungsdrücken kann hierdurch sowohl eine adäquate Blockung im Endotrachealtubus bzw. in der Trachealkanüle in der Inspiration sichergestellt werden, als auch die notwendige Entlastung für die Durchblutung der Trachealschleimhaut in der Exspirationsphase gewährleistet werden.

Der kontinuierlich gemessene Ballondruck wird auch hier als zusätzliche Druckkurve angezeigt und kann mit entsprechenden Alarmen versehen werden.

Eine automatische Hustenüberwachung kann kritische Situationen erkennen, welche ein angepasstes Druckniveau im Tubus erfordern. In dieser Situation wird in beiden Ballon-Modi ein erhöhtes Druckniveau (PBallon max) aufgebaut.

Über eine Taste der Schnittstelle kann der Anwender eine zeitweilige Überblockung des Ballons (z.B. bei Mundpflege, subglottischen Spülungen, Bauchlagerung, ...) mit einstellbarer Zeitvorgabe auslösen.

Das Manöver sowie die verbleibende Restzeit wird in der Informationszeile des Intensivventilators entsprechend angezeigt.

Auf Tastendruck kann der Ballonscout den Ballon entblocken und so die sichere Extubation erleichtern.

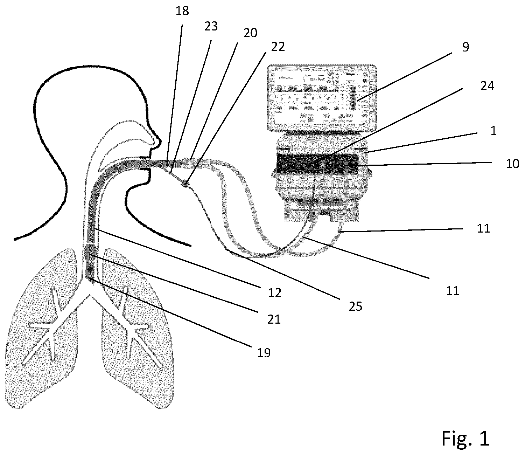

Figur 1 zeigt schematisch das System zur Atemgasversorgung umfassend eine (nicht gezeigte) Atemgasquelle 2, in einem Beatmungsgerät 1, eine Steuereinheit 3, ein Drucksteuermodul 4, ein Flusssteuermodul 6 und einen Speicher 5, wobei ebenfalls eine Drucksensoreinrichtung 7 und eine Flusssensoreinrichtung 8 und eine Anwenderschnittstelle 9 zur Vorgabe von Parametern 10 der Atemgasversorgung oder zum Austausch von Daten umfasst sind. Das System weist einen Atemgasschlauch 11 (der eine Leitung für die Inspiration und eine Leitung für die Exspiration umfassen kann) und ein Patienteninterface 12 auf. Das Beatmungsgerät 1 weist zumindest einen Schlauchanschluss 10 auf, an den der Atemgasschlauch 11 adaptiert werden kann. Das Beatmungsgerät weist auch eine Anzeige auf, zur Darstellung von Atemgaswerten oder von Atemgaskurven. Das System weist optional zusätzlich einen Anfeuchter 13 und/oder eine Sauerstoffquelle 14 und/oder einen Vernebler 15 und/oder eine Heizung 16 auf. Das Patienteninterface 12 ist beispielsweise ein Tubus für die Luftröhre oder für die Speiseröhre oder ein Blasenkatheter oder

Die Steuereinheit 3 ist eingerichtet und ausgebildet die Atemgasquelle 2, unter Verwendung des Drucksteuermoduls 4 oder des Flusssteuermoduls 6, zur Vorgabe eines Atemgasdruckes und/oder eines Atemgasflusses anzusteuern. Das Atemgas wird am Schlauchanschluss 10 bereitgestellt und über den Schlauch 11 zu einem Patienten geleitet.

Die Steuereinheit 3 ist eingerichtet und ausgebildet zusätzlich zumindest zeitweise den Anfeuchter 13 oder den Vernebler und die Heizung 16 zur Erwärmung und Anfeuchtung des Atemgases zu aktivieren.

Die Steuereinheit 3 kann zusätzlich eine Sauerstoffquelle 14 und/oder einen Vernebler 15 zur Konditionierung des Atemgases aktivieren.

Der Atemgasschlauch 11 und/oder der Anfeuchter 13 und/oder der Vernebler 15 weisen jeweils eine Heizung 16 auf. Alternativ weist zumindest der Atemgasschlauch 11 und/oder der Anfeuchter 13 und/oder der Vernebler 15 eine Heizung 16 auf.

Die Steuereinheit ist zur Erkennung von Atmungsbemühungen aus dem Drucksignal und/oder aus dem Flusssignal der Drucksensoreinrichtung und/oder der Flusssensoreinrichtung eingerichtet und ausgebildet.

Die Anwenderschnittstelle 9 zur Vorgabe von Parametern der Atemgasversorgung oder zum Austausch von Daten ist beispielsweise ausgebildet als ein Touchscreen. Alternativ oder ergänzend können auch ein Display und Eingabetasten vorgesehen sein. Ergänzend weist die Anwenderschnittstelle 9 zumindest eine Schnittstelle (USB, Bluetooth, WLAN...) zur Verbindung mit externen Geräten oder einem Netzwerk auf, um Daten auszutauschen. Vorgegebene Parameter werden im Speicher abrufbar abgelegt.

Die Atemgasquelle 2 ist beispielsweise ausgebildet als Elektromotor mit Lüfterrad oder als Druckgasquelle mit Ventilen.

Das System ist beispielsweise ausgebildet und eingerichtet, dass die Steuereinheit unter Berücksichtigung der Signale der Fluss- und/oder Drucksensoreinrichtung, eine Sauerstoffquelle zur Sauerstoffbeimischung aktiviert, die Atemphasenabhängig moduliert wird. Beispielsweise ist daran gedacht die Sauerstoffbeimischung so zu steuern, dass in Phasen der Inspiration dem Patienten mehr Sauerstoff zur Verfügung steht als zur Exspiration.

Das System ist beispielsweise ausgebildet und eingerichtet, dass ein Pulsoximeter 17 oder ein Co2-Messgerät 18 adaptiert werden kann, welches dann mit dem System kommuniziert und/oder von ihm mit Energie versorgt wird. Über die Messwerte dieser Geräte kann der Anwender - oder ein automatischer Algorithmus - die richtige Flussrate und die richtige Sauerstoff-Beimischung einstellen. Beispielsweise berücksichtigt die Steuereinheit Messwerte des Pulsoximeters 17 und/oder des Co2-Messgerätes 18 bei der Aktivierung / Steuerung der Sauerstoffquelle und/oder bei der Aktivierung / Steuerung des Flusssteuermoduls 6.

Erfindungsgemäß ist daran gedacht, dass der Anwender über die Anwenderschnittstelle eine Vorgabe an die Steuereinheit 3 eingibt das Drucksteuermodul oder das Flusssteuermodul 6 zu aktivieren. Die Vorgabe kann auch im Speicher abgelegt sein und von der Steuereinheit ausgelesen werden. Erfindungsgemäß ist auch daran gedacht, dass die Steuereinheit 3 selbststätig anhand im Speicher abgelegter Informationen das Modul 4 oder 6 wählt und unter Vorgabe der Parameter (Druckwerte, Flusswerte ...) ansteuert.

Fig. 1 zeigt auch einen Beatmungstubus 12 in der Ausführung als Endotrachealtubus mit trachealen Ende 19, mit einem distalem Ende 18 mit Tubuskonnektor 20 zum Anschluss an Beatmungsschläuche 11 für ein Beatmungsgerät 1 und einem durchgehenden Lumen für die Atemgase. Nahe am trachealen Ende 19 ist die aufblasbare Manschette 21 - auch Ballon genannt - ausgebildet, die Zuführleitung 23 zum Ballon ist nicht dargestellt.

Die Zuführleitung 23 zu dem aufblasbaren Ballon ist aussenseitig oder innenseitig am Tubus entlanggeführt oder in die Tubuswand integriert und mit einem Anschlussstecker 22 am Ende zum Anschluss 24 an das Beatmungsgerät ausgerüstet. Ein Druckschlauch 25 stellt die Verbindung her zwischen dem Druckanschluss 24 des Beatmungsgerätes und dem Anschlussstecker 22 der Zuführleitung 23.

Der Endotrachealtubus 12 ist eingeführt in die Luftröhre eines Patienten dargestellt (endend deutlich vor den Hauptbronchien).

Ein Drucksensor kann, nahe dem trachealen Ende 19, außenseitig an dem Tubus 12 befestigt bzw. in die Tubus-Wandung eingebracht und fixiert sein.

Außenseitig nahe dem trachealen Ende 19 des Tubus ist der aufblasbare Ballon 21 aus einer dünnen Kunststofffolie beispielsweise aus einem thermoplastischen Elastomer mit einer Wanddicke von beispielsweise kleiner 0,2 mm auf dem Trachealtubus befestigt.

In das Innere des Ballons führt eine Zufuhrleitung 23 für Druckgas, mit deren Hilfe der Ballon 21 mit einem Fülldruck aufgeblasen wird. Für die Zufuhr der Druckgas zu dem Ballon 21 ist beispielsweise in der Wandung des Tubus ein Kanal eingearbeitet, der im Bereich des Ballons 21 durch die Öffnung aus der Wandung des Tubus 12 austritt.

Die Steuereinheit 3 ist auch eingerichtet und ausgebildet die Atemgasquelle 2, unter Verwendung des Drucksteuermoduls 4 so anzusteuern, dass ein Druckgas über eine Leitung zum Druckanschluss 24 des Beatmungsgerätes geführt wird. Erfindungsgemäß ist zumindest ein Druckanschluss 24 vorhanden, bevorzugt zumindest zwei. Jedem Druckanschluss 24 ist ein Drucksensor zugeordnet, der den Druck des Gases in dem Druckanschluss 24 bestimmen kann. Jedem Druckanschluss 24 ist beispielsweise auch ein Ventil zugeordnet, welches eingerichtet ist, eine Druckgasströmung zu dem Druckanschluss 24 freizugeben oder zu sperren. Das Ventil wird bevorzugt über die Steuereinheit 3 gesteuert bzw. geschaltet. Das Druckgas kann der Atemgasquelle 2 entnommen werden oder einer separaten Druckgasquelle (Krankenhausleitung).

Zur Registrierung des Ballondrucks (Pbal) kann das Beatmungsgerät um ein beispielsweise in die Steuereinheit 3 integriertes Modul mit Drucksensor und Druckregler erweitert sein, welches den gewünschten Druck im Ballon einstellt, selbsttätig nachregelt und den Ballondruck (Pbal) als z. B. digitalisiertes Signal der Steuerung des Beatmungsgerätes kontinuierlich zur Verfügung stellt. Bevorzugt wird der Ballondruck (Pbal) entsprechend dem Druck der Inspiration und Exspiration variiert. Der Drucksensor kann benachbart zu oder im Druckanschluss 24 des Beatmungsgerätes angeordnet sein. Der Ballondruck (Pbal) des Tubus wird mittels des Drucksensors gemessen und die Meßwerte der Steuereinheit 3 übermittelt. Gemäß einem weiteren Vorschlag der Erfindung wird zur Optimierung der Steuerung des Beatmungsgerätes eine mit einem aufblasbaren und mit einem Fülldruck von bis zu 35 mbar beaufschlagbaren Ballon ausgerüstete Magensonde zum Einführen in die Speiseröhre des Patienten vorgesehen und der im Ballon der Magensonde herrschende ösophageale Ballondruck kontinuierlich oder intermittierend erfaßt, wobei die sich auf den Ballon der Magensonde übertragenden Schwankungen des intrathorakalen Drucks erfasst und ausgewertet werden und dem Beatmungsgerät zur Steuerung des Atemgasflusses zugeführt werden. Die in die Speiseröhre einführbare Magensonde wird durch Aufblasen ihres ösophagealen Ballons zum Anliegen an die Wandung der Speiseröhre gebracht, die in ihrem mittleren und (besser) unteren Drittel den Druckverlauf innerhalb des Brustkorbs über die Wand der Speiseröhre hinweg (transmural) auf den ösophageal platzierten Ballon der Magensonde überträgt. Der transmural übertragene Druck wird von diesem Ballon aufgenommen und als Messwert und Steuerungssignal nutzbar gemacht.

Das System ist dadurch gekennzeichnet, dass die Steuereinheit 3 eingerichtet und ausgebildet ist die Atemgasquelle 2, unter Verwendung des Drucksteuermoduls 4 oder des Flusssteuermoduls 6, zur Vorgabe eines Atemgasdruckes und/oder eines Atemgasflusses anzusteuern und das Atemgas am Schlauchanschluss 10 bereitgestellt und über den Atemgasschlauch 11 zu einem Patienten geleitet wird, wobei die Steuereinheit 3 eingerichtet und ausgebildet ist zusätzlich zumindest zeitweise den Anfeuchter 13 oder den Vernebler und die Heizung 16 zur Erwärmung und Anfeuchtung des Atemgases oder eine Sauerstoffquelle 14 zu aktivieren und wobei zusätzlich ein Pulsoximeter 17 oder ein Co2-Messgerät 18 adaptiert ist, welches dann mit dem System kommuniziert und/oder von ihm mit Energie versorgt wird und wobei die Steuereinheit 3 eingerichtet und ausgebildet ist die Messwerte dieser Geräte 17 oder 18 dem Anwender an der Schnittstelle bereitzustellen und/oder ein automatischer Algorithmus die richtige Flussrate und die Messwerte dieser Geräte 17 oder 18 für die Aktivierung oder Steuerung der Sauerstoffquelle 14 und/oder bei der Aktivierung oder Steuerung der Atemgasquelle zu nutzen.

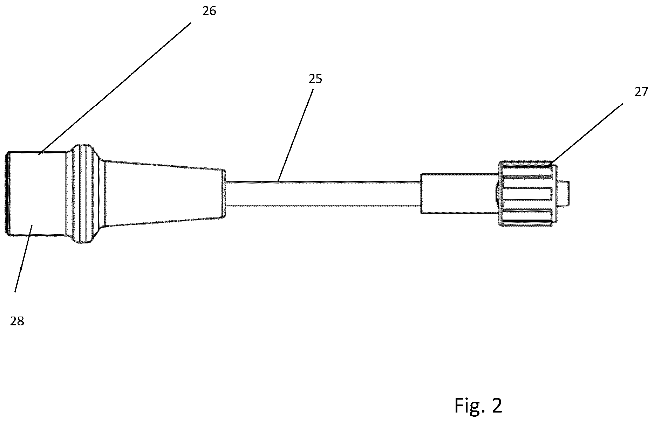

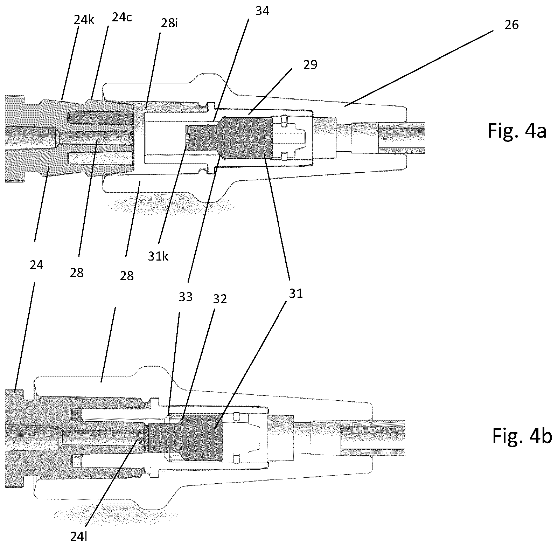

### Fig. 2 und 4 zeigen:

Der Druckadapter 26 weist einen männlichen Luer-Anschluß 27 auf, der an einem kurzen Schlauchabschnitt 25 angeordnet ist, und der zur Verbindung mit einem weiblichen Luer-Anschluss eines Druckschlauches angepasst ist.

Zusätzlich weist der Druckadapter 26 eine weibliche Aufnahme 28 für den männlichen Druckanschluss 24 des Beatmungsgerätes auf. Im Bereich der weibliche Aufnahme 28 ist ein Rückschlagventil 29 angeordnet, welches durch eine Feder 30 in einer Schließstellung 34 vorgespannt ist. Der Ventilkörper 31 des Rückschlagventils 29 kann durch den Druckanschluss 24 in eine Öffnungsstellung bewegt werden kann.

In der Schließstellung 34 (Fig. 4a) liegt die Dichtfläche 32 des Ventilkörpers 31 dichtend an der Dichtung 33 des Rückschlagventils 29 an.

Die Druckgasleitung 24l in dem Druckanschluss 24 dient auch dazu, den Ventilkörper 31 in eine Öffnungsstellung 35 (Fig. 4b) zu bewegen.

Dazu weist der Ventilkörper eine Kontaktfläche 31k auf, gegen die die (Spitze der) Druckgasleitung 24l gedrängt wird, wenn der männlichen Druckanschluss 24 in die weibliche Aufnahme 28 geschoben wird. Der männlichen Druckanschluss 24 drückt dann (mittels der Druckgasleitung 24l) den Ventilkörper 31 gegen die Federkraft in die Offenstellung 35, die einen Gasfluss aus dem männlichen Druckanschluss 24 in den Druckadpter 26 (oder den Druckschlauch 25) ermöglicht.

Die weibliche Aufnahme 28 ist bevorzugt aus einem weichen oder elastomeren Material gefertigt und weist ein Innenlumen 28i auf, welches dem Außendurchmesser 24b, c des männlichen Druckanschlusses 24 des Beatmungsgerätes so angepasst ist, dass der männlichen Druckanschluss 24 leicht in die weibliche Aufnahme 28 eingeführt werden kann, dann jedoch, wenn der männlichen Druckanschluss 24 weiter in die weibliche Aufnahme 28 eingeführt ist, eine dichtende und klemmende Verbindung zwischen beiden sicherstellt. Das Innenlumen 28i ist also kleiner als der maximale Außendurchmesser 24c.

Der Druckadapter 26 kann so auf den Druckanschluss 24 gesetzt werden und führt zu einer abgedichteten Strömungsverbindung von Druckgas aus dem Druckanschluss 24 in den Druckadapter 26.

Der neue Druckadapter 26 erlaubt somit (mittelbar über Verbindungsschläuche):
- den Anschluss eines Ösophaguskatheters an das Beatmungsgerät für ein transpulmonales Druckmonitoring und eine gastrale Druckmessung.
- den Anschluss eines Blasenkatheters an das Beatmungsgerät zur Erfassung und Überwachung des intraabdominelles Drucks.

Der neue Druckadapter 26 ermöglicht auch eine Leckageprüfung für das Beatmungsgerät, da der Druckgasfluss in den Druckadapter 26 erfolgt und dabei eine Druckmessung erfolgt. Fällt der Druck ab, wenn der Druckadapter 26 alleine, ohne Verbindungsschläuche, verwendet ist, so kann dies auf eine Leckage im Gasweg des Beatmungsgerätes hindeuten. Eine entsprechende Überwachung und Alarmierung ist durch die Steuereinheit umgesetzt.

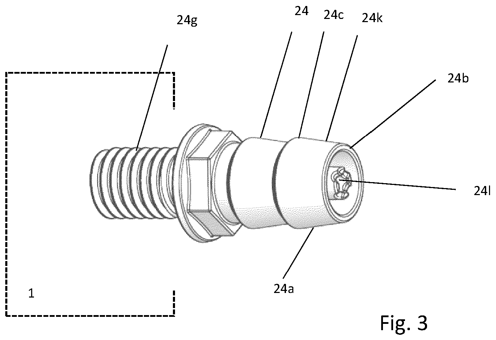

### Fig. 3 zeigt:

Der männliche Druckanschluss 24 des Beatmungsgerätes weist einen Gewindeteil 24g auf, mit dem er in das Beatmungsgerät 1 geschraubt wird, eine Druckgasleitung 24l im inneren Lumen des Druckanschlusses und einen männlichen Anschluss 24a, der zumindest einen konischen Abschnitt 24k aufweist. Der konische Abschnitt 24k weist einen geringen Außendurchmesser 24b an dem Ende 24b auf, welches dem Beatmungsgerät abgewandt ist. Der geringe Außendurchmesser 24b dient dem leichten Aufsetzen der weiblichen Aufnahme 28. Dem Beatmungsgerät weiter zugewandt nimmt der Außendurchmesser des konischen Abschnitts 24k zu und erreicht einen maximalen Außendurchmesser 24c. Im Bereich des maximalen Außendurchmessers 24c ist die weiche oder elastische weibliche Aufnahme 28 sicher fixiert.

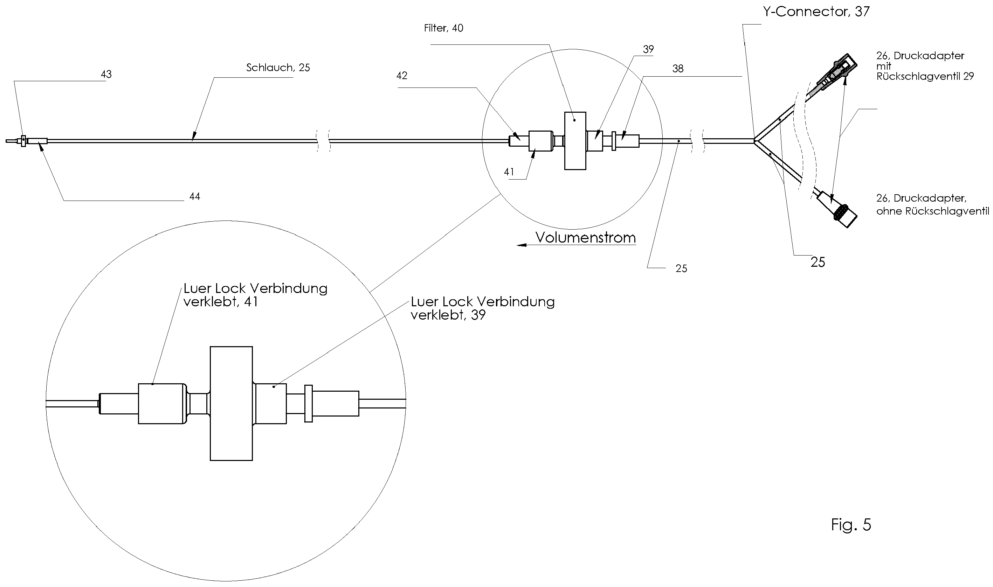

### Fig. 5 zeigt:

Ein Druckschlauch 25 stellt die Verbindung her zwischen dem Druckanschluss 24 des Beatmungsgerätes und dem Anschlussstecker 22 der Zuführleitung 23.

Dazu weist der Schlauch an einem Ende einen (männlichen) Luer-Lock Anschluss 35 auf und an dem anderen Ende zumindest einen Druckadapter 26, der auf den Druckanschluss 24 (des Beatmungsgerätes) gesetzt werden und zu einer abgedichteten Strömungsverbindung von Druckgas aus dem Druckanschluss 24 in den Schlauch und weiter in den Ballon führt.

Der zumindest eine Druckadapter 26 weist eine weibliche Aufnahme 28 für den männlichen Druckanschluss 24 des Beatmungsgerätes mit einem Rückschlagventil 29 auf, das durch eine Feder 30 in einer Schließstellung vorgespannt ist und dessen Ventilkörper 31 durch den Druckanschluss 24 in eine Öffnungsstellung bewegt werden kann.

Der Druckschlauch 25 kann ein Y-Stück 37 aufweisen, von dem zwei Druckadapter 26 abzweigen. Davon weist dann ein Druckadapter 26 ein Rückschlagventil 29 auf und der andere Druckadapter 26 weist kein Rückschlagventil auf.

Der Abschnitt des Schlauches 25 mit dem Druckadapter 26 ist über einen weiblichen Luer-Lock Anschluss 38 mit einem Filter 40 verbunden. Der Filter 40 weist dazu einen männlichen Lock Anschluss 39 auf. Der Filter 40 weist zudem einen weiblichen Luer Lock Anschluss 41 auf, an den ein männlicher Luer Lock Anschluss 42 des weiteren Druckschlauches 25 konnektiert werden kann.

Der Filter 40 weist bevorzugt einen unlösbaren, beispielsweise verklebten, männlichen Luer-Lock Anschluss 39 und einen unlösbaren, beispielsweise verklebten, weiblichen Luer-Lock Anschluss 41 auf.

Der weitere Abschnitt des Druckschlauches weist dazu einen (männlichen) Luer-Lock Anschluss 42 auf der mit dem Filter verbunden wird und einen weiteren (männlichen) Luer-Lock Anschluss 43 zur Verbindung mit dem Ballon 21. Der Luer-Lock Anschluss 43 ist über eine Schlauchtülle 44 unlösbar mit dem Schlauch 25 verbunden.

Der Druckschlauch hat folgende Einsatzmöglichkeiten:
- druckgeprüfte Sicherheitsleitung zur Verbindung zwischen Beatmungsgerät und Endotrachealtubus bzw. Trachealkanülen zur Ballonregulierung und - überwachung
- verhindert die versehentliche Entlastung des Ballons bei Dekonnektion
- erlaubt die Erfassung von Leckagen vor Aktivierung der Ballonfunktion (Prüfadapter)

## Patentansprüche

1. System zur Atemgasversorgung, umfassend ein Beatmungsgerät (1), eine Atemgasquelle (2), eine Steuereinheit (3), ein Drucksteuermodul (4) und einen Speicher (5), wobei ebenfalls eine Drucksensoreinrichtung (7), eine Flusssensoreinrichtung (8) und eine Anwenderschnittstelle (9) zur Vorgabe von Parametern der Atemgasversorgung oder zum Austausch von Daten umfasst sind, wobei das Beatmungsgerät (1) zumindest einen Schlauchanschluss (10), an den ein Atemgasschlauch (11) adaptiert werden kann, ein Patienteninterface (12) als Schnittstelle zum Patienten und zumindest einen Druckanschluss (24) für einen zusätzlichen Druckschlauch (25) aufweist, wobei als Patienteninterface (12) ein Tubus verwendet ist, der einen über den Druckanschluss (24) und den daran adaptierten Druckschlauch (25) definiert mit Druckgas befüllbaren Ballon (21) aufweist, **dadurch gekennzeichnet, dass** ein Anwender über die Anwenderschnittstelle (9) den Ballondruck festlegen kann und die Steuereinheit (3) über einen Drucksensor und einen Druckregler den gewünschten Druck im Ballon (21) einstellt und den Ballondruck (Pbal) als Signal der Steuereinheit (3) zur Verfügung stellt, wobei die Steuereinheit (3) den Ballondruck (Pbal) entsprechend dem Druck der Inspiration und der Exspiration variiert.

2. System nach Anspruch 1, **dadurch gekennzeichnet, dass** das Patienteninterface (12) als Endotrachealtubus mit einem trachealen Ende (19), einem distalen Ende (18), einem Tubuskonnektor (20) zum Anschluss an den Atemgasschlauch (11) und einem durchgehenden Lumen für die Atemgase ausgeführt ist, wobei nahe dem trachealen Ende (19) der aufblasbare Ballon (21) ausgebildet ist, wobei der Ballon (21) über eine Zuführleitung (23), die über den Druckschlauch (25) mit dem Druckanschluss (24) des Beatmungsgerätes verbunden ist, mit Druckgas aus der Atemgasquelle oder einer Druckgasquelle befüllbar ist.

3. System nach zumindest einem der vorhergehenden Ansprüche, **dadurch gekennzeichnet, dass** die Steuereinheit (3) dazu eingerichtet und ausgebildet ist, die Atemgasquelle (2) unter Verwendung des Drucksteuermoduls (4) so anzusteuern, dass Druckgas über eine Leitung zum Druckanschluss (24) des Beatmungsgerätes geführt wird, wobei zumindest ein Druckanschluss (24) vorhanden ist und jedem Druckanschluss (24) ein Drucksensor, der den Druck des Gases in dem Druckanschluss (24) bestimmen kann, und/oder ein Ventil, das dazu eingerichtet ist, eine Druckgasströmung zu dem Druckanschluss (24) freizugeben oder zu sperren, zugeordnet ist.

4. System nach zumindest einem der vorhergehenden Ansprüche, **dadurch gekennzeichnet, dass** der Druckanschluss (24) des Beatmungsgerätes ein Verbindungsmittel (24g) zur lösbaren Verbindung mit dem Beatmungsgerät, ein inneres Lumen, eine Druckgasleitung (24l) im inneren Lumen sowie einen männlichen Anschluss (24a) aufweist und ein Druckadapter (26) umfasst ist, der einen an einem kurzen Schlauchabschnitt (25) angeordneten männlichen Luer-Anschluss (27) und eine weibliche Aufnahme (28) für den männlichen Druckanschluss (24) des Beatmungsgerätes aufweist, wobei der männliche Anschluss (24a) zumindest einen konischen Abschnitt (24k) aufweist, der zur lösbaren Verbindung mit der weiblichen Aufnahme (28) des Druckadapters (26) ausgebildet ist.

5. System nach Anspruch 4, **dadurch gekennzeichnet, dass** in dem Druckadapter (26) ein Rückschlagventil (29) angeordnet ist, das in einer Schließstellung (34) vorgespannt ist und dessen Ventilkörper (31) durch den Druckanschluss (24) in eine Öffnungsstellung (35) bewegt werden kann.

6. System nach zumindest einem der Ansprüche 1 bis 3, **dadurch gekennzeichnet, dass** ein Druckschlauch (25) umfasst ist, der an einem Ende einen Luer-Lock-Anschluss (43) aufweist und an dem anderen Ende zumindest einen Druckadapter (26) aufweist, der auf den Druckanschluss (24) des Beatmungsgerätes gesteckt werden kann, wobei in dem Druckadapter (26) ein Rückschlagventil (29) angeordnet ist, das in einer Schließstellung vorgespannt ist und dessen Ventilkörper (31), bei der Verbindung mit dem Druckanschluss (24), durch den Druckanschluss (24) in eine Öffnungsstellung bewegt wird.

7. System nach Anspruch 6, **dadurch gekennzeichnet, dass** der Druckadapter (26) einen männlichen Luer-Anschluss (27) aufweist, der an einem Schlauchabschnitt (25) angeordnet ist und zur Verbindung mit einem weiblichen Luer-Anschluss des Druckschlauches angepasst ist, wobei der Druckadapter (26) zudem eine weibliche Aufnahme (28) für den männlichen Druckanschluss (24) des Beatmungsgerätes aufweist.

8. System nach Anspruch 5 oder 7, **dadurch gekennzeichnet, dass** im Bereich der weiblichen Aufnahme (28) das Rückschlagventil (29) angeordnet ist, das durch eine Feder in der Schließstellung (34) vorgespannt ist, wobei der Ventilkörper (31) des Rückschlagventils (29) durch den Druckanschluss (24) in die Öffnungsstellung bewegbar ist.

9. System nach zumindest einem der Ansprüche 5 bis8, **dadurch gekennzeichnet, dass** der Druckanschluss (24) eine Druckgasleitung (24l) aufweist, die Druckgas leitet und auch dazu dient, den Ventilkörper (31) in die Öffnungsstellung (35) zu bewegen, wenn der Druckadapter (26) mit dem Druckanschluss (24) verbunden wird.

10. System nach Anspruch 9 rückbezogen auf Anspruch 4 oder 7, **dadurch gekennzeichnet, dass** der Ventilkörper (31) eine Kontaktfläche (31k) aufweist, gegen die die Druckgasleitung (24l) gedrängt wird, wenn der männliche Druckanschluss (24) in die weibliche Aufnahme (28) geschoben wird.

11. System nach Anspruch 4 oder7, **dadurch gekennzeichnet, dass** die weibliche Aufnahme (28) aus einem weichen oder elastomeren Material gefertigt ist und ein Innenlumen (28i) aufweist, das an den Außendurchmesser (24b, 24c) des männlichen Druckanschlusses (24) des Beatmungsgerätes so angepasst ist, dass der männliche Druckanschluss (24) in die weibliche Aufnahme (28) eingeführt werden kann und eine dichtende und klemmende Verbindung zwischen beiden sichergestellt ist.

12. System nach zumindest einem der vorhergehenden Ansprüche, **dadurch gekennzeichnet, dass** die Steuereinheit (3) auch dazu eingerichtet und ausgebildet ist, die Atemgasquelle (2) unter Verwendung des Drucksteuermoduls (4) so anzusteuern, dass Druckgas über eine Leitung zum Druckanschluss (24) des Beatmungsgerätes geführt wird, wobei dem Druckanschluss (24) der Drucksensor zugeordnet ist, der den Druck des Gases in dem Druckanschluss (24) bestimmt, wobei der Druck des Gases in dem Druckanschluss (24) dem Druck im Ballon (21) (Pbal) im Wesentlichen entspricht, wenn eine druckgasleitende Verbindung zwischen dem Ballon (21) und dem Druckgasanschluss (24) hergestellt ist.

## Claims

1. A breathing gas supply system, comprising a ventilator (1), a breathing gas source (2), a control unit (3), a pressure control module (4), and a storage means (5), wherein a pressure sensor apparatus (7), a flow sensor apparatus (8), and a user interface (9) for specifying parameters of the breathing gas supply or for exchanging data are also comprised, wherein the ventilator (1) has at least one hose connection (10) to which a breathing gas hose (11) can be adapted, a patient interface (12) as the interface to the patient, and at least one pressure connection (24) for an additional pressure hose (25), wherein a tube that has a balloon (21) that can be filled with compressed gas in a defined manner via the pressure connection (24) and the pressure hose (25) adapted thereto is used as the patient interface (12), **characterized in that** a user can define the balloon pressure via the user interface (9) and the control unit (3) sets the desired pressure in the balloon (21) via a pressure sensor and a pressure regulator and provides the balloon pressure (Pbal) to the control unit (3) as a signal, wherein the control unit (3) varies the balloon pressure (Pbal) depending on the inspiratory and expiratory pressure.

2. The system according to claim 1, **characterized in that** the patient interface (12) is designed as an endotracheal tube having a tracheal end (19), a distal end (18), a tube connector (20) for connection to the breathing gas hose (11), and a continuous lumen for the breathing gases, wherein the inflatable balloon (21) is formed close to the tracheal end (19), wherein the balloon (21) can be filled with compressed gas from the breathing gas source or a compressed gas source via a supply line (23) that is connected to the pressure connection (24) of the ventilator via the pressure hose (25).

3. The system according to at least one of the preceding claims, **characterized in that** the control unit (3) is configured and designed to actuate the breathing gas source (2) using the pressure control module (4) in such a way that compressed gas is guided via a line to the pressure connection (24) of the ventilator, wherein at least one pressure connection (24) is provided and each pressure connection (24) is assigned a pressure sensor that can determine the pressure of the gas in the pressure connection (24), and/or a valve that is configured to release or block a compressed gas flow to the pressure connection (24).

4. The system according to at least one of the preceding claims, **characterized in that** the pressure connection (24) of the ventilator has a connection means (24g) for releasable connection to the ventilator, an inner lumen, a compressed gas line (241) in the inner lumen, and a male connector (24a), and a pressure adapter (26) is comprised which has a male luer connector (27) arranged on a short hose portion (25) and a female socket (28) for the male pressure connection (24) of the ventilator, wherein the male connector (24a) has at least one conical portion (24k) that is designed for releasable connection to the female socket (28) of the pressure adapter (26).

5. The system according to claim 4, **characterized in that** a non-return valve (29) which is preloaded in a closed position (34) and the valve body (31) of which can be moved through the pressure connection (24) into an open position (35) is arranged in the pressure adapter (26).

6. The system according to at least one of claims 1 to 3, **characterized in that** a pressure hose (25) is comprised which has, at one end, a luer lock connector (43) and, at the other end, at least one pressure adapter (26) that can be fitted on the pressure connection (24) of the ventilator, wherein a non-return valve (29) which is preloaded in a closed position and the valve body (31) of which is moved through the pressure connection (24) into an open position during connection with the pressure connection (24) is arranged in the pressure adapter (26).

7. The system according to claim 6, **characterized in that** the pressure adapter (26) has a male luer connector (27) that is arranged on a hose portion (25) and that is adapted for connection to a female luer connector of the pressure hose, wherein the pressure adapter (26) additionally has a female socket (28) for the male pressure connection (24) of the ventilator.

8. The system according to claim 5 or 7, **characterized in that** the non-return valve (29), which is preloaded in the closed position (34) by means of a spring, is arranged in the region of the female socket (28), wherein the valve body (31) of the non-return valve (29) can be moved through the pressure connection (24) into the open position.

9. The system according to at least one of claims 5 to 8, **characterized in that** the pressure connection (24) has a compressed gas line (241) that conducts compressed gas and also serves to move the valve body (31) into the open position (35) when the pressure adapter (26) is being connected to the pressure connection (24).

10. The system according to claim 9, referring back to claim 4 or 7, **characterized in that** the valve body (31) has a contact surface (31k) against which the compressed gas line (241) is pressed when the male pressure connection (24) is being pushed into the female socket (28).

11. The system according to claim 4 or 7, **characterized in that** the female socket (28) is manufactured from a soft or elastomer material and has an inner lumen (28i) that is adapted to the outer diameter (24b, 24c) of the male pressure connection (24) of the ventilator in such a way that the male pressure connection (24) can be introduced into the female socket (28) and a sealing and clamping connection between the two is ensured.

12. The system according to at least one of the preceding claims, **characterized in that** the control unit (3) is also configured and designed to actuate the breathing gas source (2) using the pressure control module (4) in such a way that compressed gas is guided via a line to the pressure connection (24) of the ventilator, wherein the pressure connection (24) is assigned the pressure sensor, which determines the pressure of the gas in the pressure connection (24), wherein the pressure of the gas in the pressure connection (24) substantially corresponds to the pressure in the balloon (21) (Pbal) when a compressed gas-conducting connection has been established between the balloon (21) and the compressed gas connection (24).

## Revendications

1. Système d'alimentation en gaz respiratoire, comportant un appareil respiratoire (1), une source de gaz respiratoire (2), une unité de commande (3), un module de commande de pression (4) et un réservoir (5), lequel comporte en outre un dispositif de capteur de pression (7), un dispositif de capteur de flux (8) et une interface d'utilisateur (9) pour la préconfiguration de paramètres d'alimentation en gaz respiratoire ou pour l'échange de données, dans lequel l'appareil respiratoire (1) présente au moins un raccord de tuyau (10) auquel peut être adapté un tuyau de gaz respiratoire (11), une interface de patient (12) en tant qu'interface vers le patient et au moins un raccord de pression (24) pour un tuyau de pression supplémentaire (25), dans lequel un tube peut être utilisé en tant qu'interface de patient (12), lequel présente un ballon (21) apte à être rempli de façon définie avec du gaz sous pression par le biais du raccord de pression (24) et du tuyau de pression (25) adapté à celui-ci, **caractérisé en ce qu'**un utilisateur peut fixer la pression du ballon par le biais de l'interface d'utilisateur (9) et l'unité de commande (3) règle la pression souhaitée dans le ballon (21) par le biais d'un capteur de pression et d'un moyen de réglage de pression et fournit la pression du ballon (Pbal) à l'unité de commande (3) en tant que signal, dans lequel l'unité de commande (3) modifie la pression du ballon (Pbal) en fonction de la pression de l'inspiration et de l'expiration.

2. Système selon la revendication 1, **caractérisé en ce que** l'interface de patient (12) est conçue comme un tube endotrachéal avec une extrémité trachéale (19), une extrémité distale (18), un connecteur de tube (20) pour le raccordement au tuyau de gaz respiratoire (11) et un lumen traversant pour les gaz respiratoires, dans lequel le ballon gonflable (21) est réalisé à proximité de l'extrémité trachéale (19), dans lequel le ballon (21) peut être rempli avec du gaz sous pression à partir de la source de gaz respiratoire ou d'une source de gaz sous pression par le biais d'une conduite d'amenée (23), laquelle est relié au raccord de pression (24) de l'appareil respiratoire par le biais du tuyau de pression (25).

3. Système selon l'une au moins des revendications précédentes, **caractérisé en ce que** l'unité de commande (3) est configurée et réalisée pour actionner la source de gaz respiratoire (2) à l'aide du module de commande de pression (4) de manière à guider du gaz sous pression vers le raccord de pression (24) de l'appareil respiratoire par le biais d'une conduite, dans lequel il est prévu au moins un raccord de pression (24) et à chaque raccord de pression (24) est attribué un capteur de pression apte à déterminer la pression du gaz dans le raccord de pression (24), et/ou une soupape configurée pour libérer ou bloquer un flux de gaz sous pression vers le raccord de pression (24).

4. Système selon l'une au moins des revendications précédentes, **caractérisé en ce que** le raccord de pression (24) de l'appareil respiratoire présente un moyen de raccordement (24g) pour le raccordement détachable avec l'appareil respiratoire, un lumen intérieur, une conduite de gaz sous pression (241) dans le lumen intérieur ainsi qu'un raccord mâle (24a) et comporte un adaptateur de pression (26), lequel présente un raccord Luer mâle (27) disposé au niveau d'une section de tuyau courte (25) et un logement femelle (28) pour le raccord de pression mâle (24) de l'appareil respiratoire, dans lequel le raccord mâle (24a) présente au moins une section conique (24k) réalisée pour le raccordement détachable avec le logement femelle (28) de l'adaptateur de pression (26).

5. Système selon la revendication 4, **caractérisé en ce qu'**une soupape antiretour (29) est disposée dans l'adaptateur de pression (26), laquelle est précontrainte dans une position de fermeture (34) et dont le corps de soupape (31) peut être déplacé à travers le raccord de pression (24) vers une position d'ouverture (35).

6. Système selon l'une au moins des revendications 1 à 3, **caractérisé en ce qu'**il comporte un tuyau de pression (25), lequel présente un raccord Luer Lock (43) à une extrémité et au moins un adaptateur de pression (26) à l'autre extrémité, lequel peut être enfiché sur le raccord de pression (24) de l'appareil respiratoire, dans lequel une soupape antiretour (29) est disposée dans l'adaptateur de pression (26), laquelle est précontrainte dans une position de fermeture et dont le corps de soupape (31) est déplacé à travers le raccord de pression (24) vers une position d'ouverture lors du raccordement avec le raccord de pression (24).

7. Système selon la revendication 6, **caractérisé en ce que** l'adaptateur de pression (26) présente un raccord Luer mâle (27), lequel est disposé au niveau d'une section de tuyau (25) et adapté pour le raccordement avec un raccord Luer femelle du tuyau de pression, dans lequel l'adaptateur de pression (26) présente en outre un logement femelle (28) pour le raccord de pression mâle (24) de l'appareil respiratoire.

8. Système selon la revendication 5 ou7, **caractérisé en ce que** la soupape antiretour (29) précontrainte par un ressort dans la position de fermeture (34) est disposée dans la région du logement femelle (28), dans lequel le corps de soupape (31) de la soupape antiretour (29) peut être déplacé à travers le raccord de pression (24) vers la position d'ouverture.

9. Système selon l'une au moins des revendications 5 à 8, **caractérisé en ce que** le raccord de pression (24) présente une conduite de gaz sous pression (241), laquelle guide du gaz sous pression et sert également à déplacer le corps de soupape (31) vers la position d'ouverture (35) lorsque l'adaptateur de pression (26) est relié au raccord de pression (24).

10. Système selon la revendication 9 dans la mesure où celle-ci dépend de la revendication 4 ou 7, **caractérisé en ce que** le corps de soupape (31) présente une surface de contact (31k) contre laquelle est sollicitée la conduite de gaz sous pression (241) lorsque le raccord de pression mâle (24) est poussé dans le logement femelle (28).

11. Système selon la revendication 4 ou 7, **caractérisé en ce que** le logement femelle (28) est constitué d'un matériau souple ou élastomère et présente un lumen intérieur (28i), lequel est adapté de telle façon au diamètre extérieur (24b, 24c) du raccord de pression mâle (24) de l'appareil respiratoire, que le raccord de pression mâle (24) peut être introduit dans le logement femelle (28) et qu'un raccordement étanche par serrage est assuré entre eux.

12. Système selon l'une au moins des revendications précédentes, **caractérisé en ce que** l'unité de commande (3) est également configurée et réalisée pour actionner la source de gaz respiratoire (2) à l'aide du module de commande de pression (4) de manière à guider du gaz sous pression vers le raccord de pression (24) de l'appareil respiratoire par le biais d'une conduite, dans lequel le capteur de pression déterminant la pression du gaz dans le raccord de pression (24) est attribué au raccord de pression (24), dans lequel la pression du gaz dans le raccord de pression (24) correspond essentiellement à la pression dans le ballon (21) (Pbal) lorsqu'un raccordement conducteur de gaz sous pression est établi entre le ballon (21) et le raccord de gaz sous pression (24).
